# EUROPEAN PATENT APPLICATION

(11) **EP 4 817 514 A2**
(43) Date of publication of application: **07.10.2026**
(21) Application number: 26198523.8
(22) Date of filing: 15.01.2019
(51) Int. Cl.: A61B 5/01

(54) **METHOD, APPARATUS, AND SYSTEM FOR DETECTING STAGES OF SLEEP OF PERSON**

(30) Priority: 19.01.2018 FI 20185051
(62) Document number(s) of the earlier application(s) in accordance with Art. 76 EPC: 19704849.9 / 3 740 125
(71) Applicant: Night Train Oy, 90420 Oulu (FI)
(72) Inventor: Rytky, Juha, 90420 Oulu (FI); Myllyoja, Kimmo, 90520 Oulu (FI); Pylkko, Heikki, 54800 Savitaipale (FI)
(74) Representative: LEITZINGER OY

(57) **Abstract**

The present disclosure describes a method and an apparatus for detecting sleep stages of a person by a computer-implemented detection system comprising a detection unit and a memory, the method comprising: receiving, via wireless communication, first, second and third measurement data from a wearable device separate from the detection unit, the first, second and third measurement data being obtained by a distal skin temperature sensor, a heart rate sensor and an acceleration sensor, respectively; wherein the first measurement data represents distal skin temperature and/or distal skin temperature variability, the second measurement data represents heart rate and/or heart rate variability (HRV), and the third measurement data represents physical activity, wherein variability represents dispersion of measurement values over time; determining temperature variability and HRV over time based on the first and second measurement data; and detecting sleep-stage instances based on the temperature variability, HRV and physical activity, wherein detecting comprises: i) comparing the temperature variability with a predetermined temperature variability limit; ii) when the temperature variability exceeds the limit, determining light sleep or arousal based on comparison of physical activity with a physical activity limit; and iii) when the temperature variability is at or below the limit, determining REM sleep or deep sleep based on comparison of HRV with a heart rate variability limit.

## Description

The invention relates to monitoring sleep of a person, and in particular, to detecting stages of sleep of the person.

### BACKGROUND INFORMATION

Sleep of a human follows a circadian rhythm, i.e. a sleep-wakefulness cycle that is on average 24.09 ± 0.2 h (24 h 5 min ± 12 min) for women and 24.19 ± 0.2 h (24 h 11 min ± 12 min) for men, aged 18 to 74 years. The circadian rhythm is typically synchronized to the day-night cycle, i.e. the light-darkness cycle. During sleep within the circadian rhythm, a natural sleep cycle repeats itself at a constant interval reflecting the 1-to-2-hour ultradian basic rest-activity cycle. The natural sleep cycle comprises a plurality of stages that repeat during the sleep. These stages include REM (Rapid Eye Movement) sleep and non-REM (NREM) sleep, which can be further divided in to light sleep and deep sleep.

An adult person may be considered to need on average a minimum of 4 full sleep cycles that are guided by the human circadian system controlling and the sleep-wake cycle. Less sleep may result in various negative effects to wellbeing and health. In order to ensure sufficient amount of sleep for a person, it may be desirable to try to monitor the sleep and to estimate the progress of the person's natural sleep cycles.

Various attempts have been made to estimate the progress of the natural sleep cycles, based on the nocturnal movements of a person, for example. However, there is no scientific evidence that the movements correlate with the sleep stages within the sleep cycle. Therefore, measurement of nocturnal movements of a person is not a reliable information source for tracking the sleep stages of the person.

### BRIEF DISCLOSURE

An object of the present disclosure is to provide a method and an apparatus for implementing the method so as to alleviate the above disadvantages. The object of the disclosure is achieved by a method and apparatus which are characterized by what is stated in the independent claims. The preferred embodiments of the disclosure are disclosed in the dependent claims.

The present disclosure describes a method for detecting stages of sleep of a person. During REM (Rapid Eye Movement) sleep stage and deep sleep stage of a person's sleep, the person's body temperature control effectively shuts off and distal skin temperature changes on the limbs may be within just about 0,2 degrees Celsius. During the other sleep stages the human's temperature control is active and the distal skin temperature changes are much larger.

These two stages of sleep are therefore distinguishable from other stages of sleep and arousal (i.e. state of being awoken) of the person. However, it is difficult to distinguish these two stages from each other based on the temperature.

Heart rate variability (HRV) is another parameter that is affected by different stages of sleep. Importantly, REM sleep and deep sleep show different HRV characteristics. Therefore, it is possible to use HRV for distinguishing REM sleep and deep sleep from each other. By combining these two measurements (temperature variability and HRV), different stages of sleep can be identified.

The method according to the present disclosure provides accurate and cost-efficient means for detecting different stages of sleep. The measurements can be performed in a non-invasive manner that is more comfortable to the user. The method can be used for obtaining important information (e.g. ratios of different stages of sleep, length of sleep cycle, and circadian rhythm) on the sleep of the user.

### BRIEF DESCRIPTION OF THE DRAWINGS

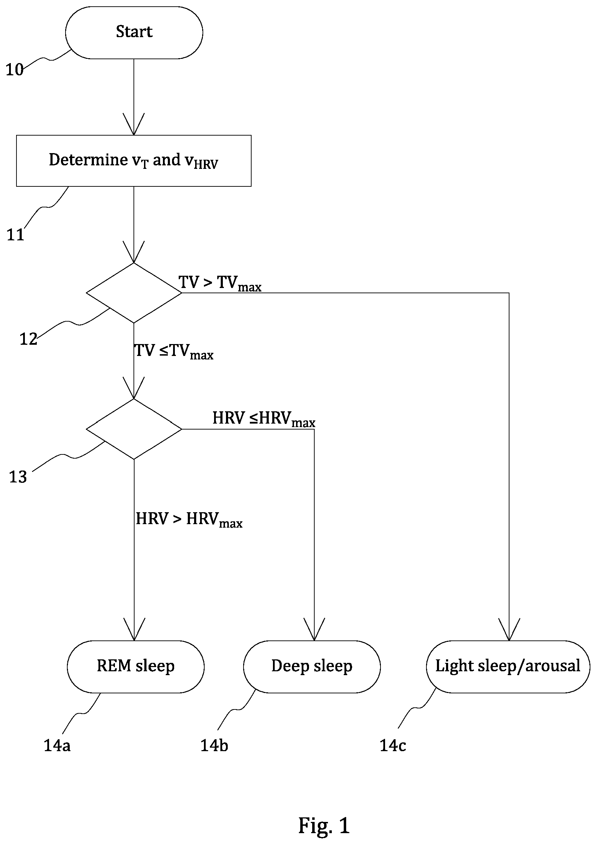

In the following the invention will be described in greater detail by means of preferred embodiments with reference to the attached drawings, in which
Figure 1 shows a simplified diagram of an exemplary embodiment of the method according to the present disclosure.

### DETAILED DISCLOSURE

The present disclosure describes a method for detecting stages of sleep of a person. A method according to the present disclosure comprises receiving temperature data and heart rate data of the person, determining temperature variability on the basis of the temperature data, determining heart rate variability (HRV) on the basis of the heart rate data, and detecting stages of sleep of the person on the basis of the temperature variability and heart rate variability. In the context of the present disclosure, the term "variability" represents dispersion (scatter, spread) of the measurements over time. The variability may be calculated as variance, standard deviation, and interquartile range of the measurements, for example.

The method may detect that the person is in a REM (Rapid Eye Movement) sleep stage when the temperature variability is below a first temperature variability limit and the heart rate variability is above a first heart rate variability limit. In other words, the method may comprise comparing the temperature variability with the first temperature variability limit, comparing the heart rate variability with the first heart rate variability limit, and considering that the person is in the REM sleep stage when a first condition is detected, the first condition being that the temperature variability is below the first temperature variability limit and the heart rate variability is above the first heart rate variability limit.

In addition or alternatively, the method may detect that the person is in a deep sleep stage when the temperature variability is below a second temperature variability limit and the heart rate variability is below a second heart rate variability limit. In other words, the method may comprise comparing the temperature variability with the second temperature variability limit, comparing the heart rate variability with the second heart rate variability limit, and considering that the person is in the deep sleep stage when a second condition is detected, the second condition being that the temperature variability is below the second temperature variability limit and the heart rate variability is below the second heart rate variability limit.

Figure 1 shows a simplified diagram of an exemplary embodiment of the method according to the present disclosure. In Figure 1, the first temperature variability limit is the same as the second temperature variability limit, and the first heart rate variability limit is the same as the second variability limit.

The method in Figure 1 starts from step initial step 10 from which the method moves to step 11. In step 11, the temperature variability TV and the heart rate variability HRV are determined and the method moves to step 12.

In step 12, the temperature variability TV is compared with a predetermined temperature variability limit TVmax. If temperature variability TV exceeds the temperature variability limit TVmax, the stage of sleep is considered to be light sleep stage or arousal and the method continues to a final step 14c identifying the sleep stage as light sleep or arousal. However, if temperature variability TV the same or less than temperature variability limit TVmax, the method moves to step 13.

In step 13, the heart rate variability HRV is compared with a predetermined heart rate variability limit HRVmax. If heart rate variability HRV exceeds the heart rate variability limit HRVmax, the stage of sleep is considered to be REM sleep and the method continues to a final step 14a identifying the sleep stage as REM sleep. However, if heart rate variability HRV the same or less than the heart rate variability limit HRVmax, the stage of sleep is considered to be REM sleep and the method continues to a final step 14b identifying the sleep stage as deep sleep.

A person has typically several brief periods of arousals during his/her nightly sleep. During these periods, the person is typically more restless, which can be detected as more physical activity. Therefore, in some embodiment, the method may further comprise measurement of physical activity (i.e. actigraphy). The measurement of physical activity of the person may be estimated based on an acceleration sensor attached to the person, for example. The measurement of physical activity can then be used for distinguishing the periods of arousal from the cycles of sleep. For example, in step 14c of Figure 1, arousal may be distinguished from light sleep based on the physical activity. The level of physical activity may be is compared with a predetermined physical activity limit, and if the level exceeds the limit, the stage of sleep may be considered to be arousal. If the level of physical activity does not exceed the limit, the stage of sleep may be considered to be light sleep.

Although Figure 1 uses only one limit for temperature variability and one limit for heart rate variability, the method according to the present disclosure may also utilize different temperature variability limits for REM sleep, deep sleep and light sleep/arousal. Alternatively or in addition, the method according to the present disclosure may also utilize different heart rate variability limits for REM sleep, deep sleep and light sleep/arousal.

With the method according to the present disclosure, important information on the quality of the sleep of a person can be obtained. Based on the detected stages of sleep, estimates of ratios of different stages of sleep during night can be calculated, for example. The ratios may be represented as percentages or amount (e.g. in minutes) of total sleep during the night, for example.

Another important parameter of sleep of a person is the interval (i.e. length) of a natural sleep cycle of the person. Typically, the interval (i.e. length) of a natural sleep cycle is approximately 90 minutes for an adult, but it may vary between individuals. For example, depending on the age of the person, the length of the sleep cycle may vary between 40 minutes to 130 minutes. However, within a time frame of a day or several days, the interval at which the natural sleep cycle repeats itself may be considered to remain rather constant. An adult person may be considered to need 4 to 6 full sleep cycles.

With the method according to the present disclosure, the interval between the occurrences of (or starts of) sleep cycles of specific type (e.g. REM sleep cycles or deep sleep cycles) can be detected. By determining the interval between these the sleep cycles, an estimate of the length of the natural sleep cycle of the person can be calculated. For example, a good estimate of the length of the sleep cycle may be calculated on the basis of an interval between the starts of the second and third REM sleep cycle after beginning of sleep. The estimate of the length of the sleep cycle may be based on an average of intervals between subsequent sleep cycles of the same type, for example.

Information on the length and contents of the natural sleep cycle can be coupled with the circadian rhythm, and in particular with melatonin onset (start of nocturnal melatonin production). There is a direct link between the melatonin onset and the circadian rhythm. The ability to fall asleep is linked to the melatonin onset. Thus, the melatonin onset provides a fixed reference point within the circadian rhythm of the person. The melatonin onset is observable as a change in the body temperature of the person. An estimate of the melatonin onset can be detected with a distal skin temperature measurement and an actigraphy measurement, for example. At the beginning of sleep of a person, his/her distal skin temperature rises rapidly for a while. However, at the same time the person typically remains relatively still which can be detected as a low actigraph value, e.g. in the form of low value (e.g. a value below a predetermined limit) of measurement data from an acceleration sensor.

Within the context of the present disclosure, temperature of a person may be measured in various ways. For example, the temperature samples may originate from a temperature sensor arranged to measure the distal skin temperature. In this context, distal skin temperature refers to skin temperature at extremities of body, such as the limbs and the head of a person. For example, distal skin temperature may be measured from a finger, wrist, ankle, forehead or ear lobe. The distal skin temperature may be measured with a MEMS-based temperature sensor or an infrared sensor, for example. In addition, the above-discussed phenomenon (i.e. change in the temperature variability) is detectable also in the core body temperature of the person albeit in smaller magnitudes. However, optical heart rate monitoring (e.g. pho-toplethysmography (PPG)) may be used to determine an estimate of relative core body temperature of the person. Further, an estimate of the heart rate may be extracted from graphical data provided by a digital video camera recording video of a portion of person's skin. Based on this estimate, variability of the relative temperature may be calculated, and changes in the variability of the relative temperature may then be used to detect the different stages of sleep and the melatonin onset. The temperature sensor may be a part of a wearable device, for example. The temperature sensor may also be integrated to a vehicle, e.g. in the form of a video camera recording a view on the face of the driver of a car, and the method according to the present disclosure may be used for estimating the driver's alertness.

In order to be able to detect changes in the variability of the temperature, the temperature has to be sampled at a sufficient frequency. For example, the distal temperature may be sampled at least once a minute, e.g. once every 30 seconds. The temperature may be periodically sampled and the samples may be stored. Samples may be stored for one night or over a longer period, ranging from a plurality of days to a plurality of weeks, for example. Based on the samples, variability of the temperature may then be calculated by using known algorithms, for example. In case of longer measurement periods and larger quantities of data, the data may be sent from a wearable device making the measurements to a larger data storage, such as a computer server or a computing cloud, for example.

Information on detected stages of sleep may be utilized in various ways. For example, in a first embodiment of a method according to the present disclosure, the method comprises calculating ratios of different stages of sleep of the person based on the basis of the detected stages of sleep. This kind of information can provide an important insight to the quality of the sleep. The quality of sleep can have a significant effect on the wellbeing and health of a person. Thus, the method may be configured to provide information of ratios of different stages of sleep to the user. This information may then be utilized in estimating the quality of the sleep of the user, for example.

The detected stages may also be used for determining an optimal time window for sleep. In a second embodiment of a method according to the present disclosure, the method may comprise estimating an optimal instant for a wake-up for a person on the basis of the detected stages of sleep. If a wake-up (e.g. in the form of an alarm) is scheduled to occur at a light sleep stage within the natural sleep cycle, the person wakes up feeling fresh and energetic. Calculation of an optimal wake-up instant may comprise determining the length of a sleep cycle of the person, calculating the number of specific sleep stages already observed during night's sleep on the basis of the detected stages of sleep, and determining optimal wake-up instant on the basis of the length of the sleep cycle and the number of sleep stages already observed. For example, one or more lengths of the sleep cycle may be added to the last observed sleep stage of a specific type in order to determine an optimal wake-up instant so that the person will have slept a desired number (e.g. 4, 5, or 6) of sleep cycles at the optimal wake-up instant. Once the optimal instant has been reached, an alarm may be raised in order to wake up the person. In embodiments where the physical activity of the person is measured, an increased physical activity may be used as an indication of waking up from the sleep. For example, a detected increase the physical activity that is above a predetermined detection limit for a set amount of time may be used as an indicator that the alarm has performed its task.

In addition or alternatively in the second embodiment, estimates of optimal instants of wake-up may also be calculated beforehand on the basis of the length of the natural sleep cycle and a reference point within the circadian rhythm of the person, such as the melatonin onset. Thus, the method may comprise determining a length of a sleep cycle of the person, determining a fixed reference point within a circadian rhythm of the person, and determining the optimal wake-up instant on the basis of the length of the sleep cycle and the fixed reference point so that the person will have slept a desired number of sleep cycles at the optimal wake-up instant. For example, an optimal wake-up instant may be calculated by adding a plurality (e.g. 4, 5, or 6) of determined lengths of the natural sleep cycle to the time of a known time instant of the melatonin onset. Once the optimal instant has been reached, an alarm may be raised in order to wake up the person.

In addition or alternatively, the method according to the present disclosure may also be used for determining an estimate of an optimal instant for turning in for sleep. According to a third embodiment of the method according to the present disclosure, the method may determine the optimal sleep onset (i.e. an optimal instant for turning in for sleep) based on a reference point within the circadian rhythm of the person. The reference point may represent the melatonin onset, for example. The method may calculate the next optimal instant for a sleep onset based on the length of the sleep cycle, in case the person misses any preceding optimal instants for sleep onset.

In embodiments where distal skin temperature is measured, detection of a specific temperature change pattern within distal skin temperature data may be utilized in detecting the melatonin onset. A rapid, distinct pattern in the distal skin temperature can be observed after the nocturnal melatonin production has started. The temperature change pattern that is in the form of a drop in the distal skin temperature followed an increase in the distal skin temperature, wherein the drop and the increase occur within a time window of ten minutes or less, for example five minutes. The magnitude of the increase is higher than the magnitude of the drop. The magnitude of the drop may be approximately 0.5 °C and the magnitude of the increase may be approximately 1.5 °C, for example. When the characteristic temperature change pattern is observed in the distal temperature of a person, he or she has approximately 20 to 30 minutes to get to sleep. Thus, the temperature change pattern serves as a fixed reference point within the circadian rhythm. Once a person has fallen asleep, the temperature change patterns cease to occur. The temperature change patterns will re-start again if the person awakes in the middle of the night. This may be used as a further indicator of sleep when monitoring the sleep of a person. When she or he falls asleep, the distal skin temperature starts to increase constantly by about 2 °C in the forthcoming 120 minutes. By determining the interval between the occurrences of the temperature change patterns, another estimate of the length of the natural sleep cycle of the person can be calculated.

This additional information may be used for providing additional/alternative estimates on the circadian rhythm, the length of the sleep cycle, and the stages of the natural sleep cycle during the circadian rhythm. Certainty and accuracy of a detection method according to the present disclosure may be improved by combining the detection of stages of sleep and the detection of the temperature patterns. Alternatively, in some embodiments, the length of the sleep cycle may be based solely on the temperature change patterns. For example, the length of sleep cycle in the second embodiment discussed above may be based on the interval between temperature change patterns instead of the intervals between the detected sleep stages. The length of sleep cycle may also be based on the interval between temperature change patterns in the third embodiment above.

The accuracy of the estimate of the circadian rhythm and the length of the sleep cycle may be further improved by gathering data for a plurality of days and by using this data for calculating the estimate. In some embodiments, a method according to the present disclosure may further comprise monitoring movements of the monitored person in order to improve the accuracy of the determination of the sleep cycle and the circadian rhythm.

The present disclosure further discloses a detection unit and a system for detecting stages of sleep of a person. The detection unit may comprise means configured to receive samples of temperature and heart rate of the person, and carry out the steps of an above-described method according to the present disclosure. The detection unit may be a stand-alone apparatus or it may be a part of a larger system. For example, the detection unit may be a stand-alone apparatus that comprises both sensors for measuring a distal skin temperature and heart rate of a person, and means for implementing the method for detecting stages of sleep of a person according to the present disclosure. The apparatus may comprise control unit comprising a computing device (such as a processor, an FPGA, or an ASIC) and a memory which may act as the means for implementing the method. The stand-alone apparatus may be in the form a wearable device, such as an activity bracelet or a heart rate monitor, that comprises a temperature sensor for monitoring a distal skin temperature of the person. The control unit in the wearable device may be configured to receive samples of the measured temperature (e.g. distal skin temperature) and heart rate from the sensors and detect the stages of sleep based on the samples. The control unit may be configured to calculate percentages or amount (e.g. in minutes) of different stages of sleep based on the sample, and the wearable device may comprise a display on which the calculated percentages may be shown. The control unit of the wearable device may also be configured to estimate an optimal instant for a wake-up and/or an optimal instant for turning in for sleep on the basis of the detected stages of sleep. The wearable device may implement a timer or clock function, and once an optimal instant has been reached, the wearable device may cause an alarm to be raised, in the form of an audible or visual cue, for example. The wearable device may be configured to implement also other functionalities in addition to a method according to the present disclosure.

In some embodiments, the detection unit may also be a part of a larger detection system. For example, a detection system may comprise a separate measurement unit comprising a distal skin temperature sensor and a heart rate sensor and a separate detection unit that configured to receive samples of distal skin temperature and heart rate from the measurement unit. Alternatively or in addition, the measurement unit may provide the detection unit with temperature variability and/or heart rate variability data calculated on the basis of the distal skin temperature and heart rate data.

For simplicity, temperature data and temperature variability data may be jointly referred to as first data in the context of the present disclosure. Similarly, heart rate data and heart rate variability (HRV) data may be referred to as second data.

The measurement unit may comprise a wireless communication unit through which the temperature and heart rate data is sent to the separate detection unit. The wireless connection unit may transmit the temperature and heart rate data via Blue-tooth, ZigBee, near field communication (NFC), or infrared protocols, for example. The measurement unit may be in the form of an activity tracker, a smartwatch, an earbud, a ring, an e-sticker (i.e. an adhesive sensor), for example. The separate detection unit may be a generic computing device or system, for example. A method according to the present disclosure may be implemented in the form of a computer program product having instructions which, when executed by the computing device or system, cause the computing device or system to perform a method according to the present disclosure, also including the first, second, and third embodiment described above. For example, the separate detection unit may be a handheld communication device, such as a smart phone or a tablet computer or even infotainment device or any kind of medical device, to which the computer program product is downloaded. The handheld communication device acting as the separate detection unit may be configured to wirelessly receive temperature and heart rate data and/or temperature variability and heart rate variability data originating from the measurement unit and detect and display sleep quality information, such as information on stages of sleep, on the basis of the data. The communications device may also determine an optimal time window for sleep of the person on the basis of the received data. The handheld communication device may be configured to estimate an optimal instant for a wake-up and/or an optimal instant for turning in for sleep on the basis of the determined optimal time window for sleep, and raise an alarm on the basis of the estimated optimal instant. The handheld-communication device may utilize personal preferences of the user stored in the device in the estimation of the optimal time window for sleep.

Alternatively, cloud computing may be utilized for implementing a method according to the present disclosure, also including the first, second, and third embodiment described above. For example, a cloud computing system acting as a separate detection unit may configured to receive temperature and heart rate data and/or temperature variability and heart rate variability data of a person, detect the stages of sleep and/or temperature change patterns on the basis of the data, and determine sleep quality information and/or an optimal time window for sleep of the person. The cloud computing system may be configured to estimate an optimal instant for a wake-up and/or an optimal instant for turning in for sleep on the basis of the determined optimal time window for sleep, and cause an alarm to be raised on the basis of the estimated optimal instant. For example, the cloud computing system may send an indication to a handheld communication device that an alarm should be raised. The handheld communication device then raises an alarm on the basis of this indication.

Functionalities of the method according to the present disclosure may also be divided between a cloud computing system and a separate decision-making unit. For example, the cloud may detect the stages of sleep and/or temperature change patterns as described above, and send information on the detected stages of sleep and/or temperature change patterns to a decision-making unit that estimates the quality of sleep and/or calculate and/or determines the optimal time window for sleep on the basis of the timing information. The decision-making unit may be a handheld communication device, for example. The decision-making unit may be further configured to calculate an optimal instant for a wake-up and/or an optimal instant for turning in for sleep, and raise an alarm on the basis of the estimated optimal instant based on personal preferences of the user stored on the decision-making unit.

The method (and a detection unit and system implementing the method) according to the present disclosure may be utilized in various applications. For example, in addition to the examples above, the method according to the present disclosure may also be utilized in minimizing jet lag. For a human, it is possible to adjust the circadian rhythm approximately by one hour per day. With the method according to the present disclosure, this adjustment can be done in a systematic manner.

In addition, since muscle recovery after exercise is closely related to the ratio of deep sleep within the total sleep, the information on ratios of different stages of sleep may be utilized when determining an optimal window of sleep for an athlete so s/he will have sufficient deep sleep for muscle recovery, for example. Further, humans need a sufficient time of REM sleep in order to be assimilate cognitive and motoric skills that are very essential skills e.g. for athletes. Recovery algorithms are based on the HRV and they can be significantly improved by using the quantity and quality of sleep. Further, medical personnel may utilize the information when determining the symptoms and cure of a patient in case of depression and sleep apnea, for example.

Examples 1 to 10 below describe some referential embodiments of detecting stages of sleep of a person within the context of the present disclosure.
Example 1. A method for detecting stages of sleep of a person, wherein the method comprises
   receiving temperature data and heart rate data of the person,
   determining temperature variability on the basis of the temperature data and determining heart rate variability (HRV) on the basis of the heart rate data,
   detecting stages of sleep of the person by
      i) detecting that the person is in a REM (Rapid Eye Movement) sleep stage when the temperature variability is below a first temperature variability limit and the heart rate variability is above a first HRV limit, and/or
      ii) detecting that the person is in a deep sleep stage when the temperature variability is below the first temperature variability limit and the heart rate variability is below a second HRV limit.
Example 2. A method according to Example 1, wherein the method further comprises
   calculating ratios of different stages of sleep of the person based on the detected stages of sleep.
Example 3. A method according to Example 1 or 2, wherein the method further comprises
   estimating an optimal instant for a wake-up and/or an optimal instant for turning in for sleep on the basis of the detected stages of sleep.
Example 4. A method according to Example 3, wherein the method comprises
   determining a length of a sleep cycle of the person,
   calculating a number of specific sleep stages already observed during night's sleep on the basis of the detected stages of sleep, and
   determining the optimal wake-up instant on the basis of the length of the sleep cycle and the number of sleep stages already observed so that the person will have slept a desired number of sleep cycles at the optimal wake-up instant.
Example 5. A method according to Example 3, wherein the method comprises
   determining a length of a sleep cycle of the person,
   determining a fixed reference point within a circadian rhythm of the person,
   determining the optimal wake-up instant on the basis of the length of the sleep cycle and the fixed reference point so that the person will have slept a desired number of sleep cycles at the optimal wake-up instant.
Example 6. A method according to any one of Examples 1 to 5, wherein the method further comprises
   measuring temperature data and pulse data of the person with a measurement band or bracelet worn by the person.
Example 7. A method according to Example 5, wherein the determining of the reference point of a circadian comprises
   detecting a temperature change pattern in the samples of distal skin temperature, wherein the temperature change pattern indicates a reference point for an optimal time window for sleep, wherein the temperature change pattern is in the form of a drop in the distal skin temperature followed by an increase in the distal skin temperature, where the drop and the increase occur within a time window of ten minutes or less.
Example 8. A method according to Example 7, wherein a magnitude of the drop is approximately 0.5 °C and a magnitude of the increase is approximately 1.5 °C.
Example 9. A detection unit comprising means configured to carry out a method according to any one of Examples 1 to 8.
Example 10. A detection unit according to Example 9, wherein the detection unit is a wearable device in the form of band or bracelet comprising
   a temperature sensor,
   a heart rate sensor, and
   a control unit configured to carry out a method according to any one of Examples 1 to 8.

It is obvious to a person skilled in the art that the method and system according to the present disclosure can be implemented in various ways. The invention and its embodiments are not limited to the examples described above but may vary within the scope of the claims.

## Claims

1. A method for detecting stages of sleep of a person by a detection system comprising a detection unit, the detection unit being computer-implemented with a computing device or a system with a computer program product and a memory,
wherein the method comprises:
receiving automatically a first measurement data, a second measurement and a third measurement data transmitted wirelessly by a wireless communication unit from a measurement unit in a form of wearable device separated from the detection unit from a distal skin temperature sensor, a heart rate sensor and an acceleration sensor of the wearable device; wherein the first measurement data represents at least one of distal skin temperature and distal skin temperature variability of the person, and the second measurement data represents at least one of heart rate and
heart rate variability of the person, and the third measurement data represents physical activity of the person, wherein variability represents dispersion of measurement values over time;
determining temperature variability of the person over time on the basis of the temperature data; and determining heart rate variability (HRV) of the person over time on the basis of the heart rate data;
detecting instances of sleep stage within sleep of the person based on the distal skin temperature variability over time and the heart rate variability over time, and
the physical activity, wherein the detecting of the instances of the sleep stages comprises:
i) comparing the distal skin temperature variability to a predetermined temperature variability limit; ii) in response to the distal skin temperature variability exceeding the predetermined temperature variability limit, further detecting whether the person is in a light sleep stage or an arousal stage by comparing the physical activity to a physical activity limit; and iii) in response to the distal skin temperature variability being at or below the predetermined temperature variability limit, further detecting whether the person is in a Rapid Eye Movement (REM) sleep stage or a deep sleep stage by comparing the heart rate variability to a heart rate variability limit.

2. A method according to claim 1, wherein the method further comprises:
estimating an optimal instant for a wake-up and/or an optimal instant for turning in for sleep based on the detected stages of sleep.

3. A method according to claim 2, wherein the method comprises:
determining a length of a sleep cycle of the person, and
estimating the optimal wake-up instant based on the length of the sleep cycle so that, after a night's sleep, the person will have slept a desired number of sleep cycles at the optimal wake-up instant.

4. A method according to claim 2, wherein the method comprises:
determining a length of a sleep cycle of the person,
determining a fixed reference point within a circadian rhythm of the person,
estimating the optimal wake-up instant based on the length of the sleep cycle and the fixed reference point so that the person will have slept a desired number of sleep cycles at the optimal wake-up instant.

5. A method according to any one of claims 2 to 4, wherein the estimating of the optimal instant for a wake-up or the optimal instant for turning in for sleep further comprises:
gathering measurement data for a plurality of days,
calculating an estimate of the circadian rhythm of the person based on the gathered data, and
estimating the optimal instant for a wake-up and/or the optimal instant for turning in for sleep based on the detected stages of sleep and the estimate of the circadian rhythm.

6. A method according to any one of the preceding claims, wherein the providing of the information on quality of the sleep comprises:
calculating ratios of different stages of sleep of the person based on the detected stages of sleep, and
using at least the calculated ratios as the information on the quality of sleep.

7. A detection system for monitoring sleep of a person, the detection system comprising:
a detection unit, the detection unit being computer-implemented with a computing device or a system with a computer program product and a memory; and
a wearable device with a distal skin temperature sensor, a heart rate sensor and an acceleration sensor, the wearable device being separate from the detection unit and transmitting data wirelessly to the detection unit, the wearable device being a measurement unit;
the detection unit configured to automatically:
receive first measurement data, second measurement and third measurement data wirelessly through a wireless communication unit from the distal skin temperature sensor, the heart rate sensor and the acceleration sensor of the wearable device,
wherein the first measurement data represents at least one of distal skin temperature and distal skin temperature variability of the person, and the second measurement data represents at least one of heart rate and heart rate variability of the person, and the third measurement data represents physical activity of the person,
wherein variability represents dispersion of measurement values over time,
determine distal skin temperature variability of the person over time based on the first measurement data and determining heart rate variability of the person over time based on the second measurement data,
detect instances of sleep stage within sleep of the person based on the distal skin temperature variability over time and the heart rate variability over time, and the physical activity, wherein the detecting of the instances of the sleep stages comprises:
i) comparing the distal skin temperature variability to a predetermined temperature variability limit; ii) in response to the distal skin temperature variability exceeding the predetermined temperature variability limit, further detecting whether the person is in a light sleep stage or an arousal stage by comparing the physical activity to a physical activity limit; and iii) in response to the distal skin temperature variability being at or below the predetermined temperature variability limit, further detecting whether the person is in a Rapid Eye Movement (REM) sleep stage or a deep sleep stage by comparing the heart rate variability to a heart rate variability limit.

8. A detection system according to claim 7, wherein the detection system is further configured to
estimate an optimal instant for a wake-up and/or an optimal instant for turning in for sleep based on the detected stages of sleep.

9. A detection system according to claim 8, wherein the estimating of the optimal instant for a wake-up or the optimal instant for turning in for sleep comprises:
determining a length of a sleep cycle of the person, and
estimating the optimal wake-up instant based on the length of the sleep cycle so that, after a night's sleep, the person will have slept a desired number of sleep cycles at the optimal wake-up instant.

10. A detection system according to any one of claims 7 to 9, wherein the estimating of the optimal instant for a wake-up or the optimal instant for turning in for sleep further comprises:
gathering measurement data for a plurality of days,
calculating an estimate of the circadian rhythm of the person based on the gathered data, and
estimating the optimal instant for a wake-up and/or the optimal instant for turning in for sleep based on the detected stages of sleep and the estimate of the circadian rhythm.

11. A detection system according to any of the claims 7 to 10, wherein the detection system further comprises a measurement unit in the form of a wearable device, such as a ring, an activity tracker, a smartwatch, an earbud, an e-sticker or a band, comprising at least:
a temperature sensor configured to measure distal skin temperature,
a heart rate sensor configures to measure heart rate, and
an acceleration sensor configured to measure physical activity.
